# EUROPEAN PATENT APPLICATION

(11) **EP 4 844 068 A1**
(43) Date of publication of application: **25.11.2026**
(21) Application number: 25808124.9
(22) Date of filing: 23.05.2025
(51) Int. Cl.: C12N 15/77, C12N 9/12, C12P 13/02, C12P 7/42, C12R 1/15

(54) **MICROORGANISM HAVING INCREASED ACTIVITY OF LIPOATE PROTEIN LIGASE, AND USE THEREOF**

(30) Priority: 24.05.2024 KR 20240067864
(71) Applicant: CJ Cheiljedang Corporation, Seoul 04560 (KR)
(72) Inventor: SHIN, Kwang Soo, Seoul 04560 (KR); JANG, Yeon-Jae, Seoul 04560 (KR); SEO, Chang Il, Seoul 04560 (KR)
(74) Representative: Meissner Bolte Partnerschaft mbB
(86) International application number: PCT/KR2025/007006
(87) International publication number: WO 2025/244453

(57) **Abstract**

The present disclosure provides a microorganism with increased activity of lipoate protein ligase, a composition for producing pantothenic acid and/or pantoic acid comprising the microorganism, and a method for producing pantothenic acid and/or pantoic acid comprising a step of culturing the microorganism, wherein the microorganism has excellent pantothenic acid and/or pantoic acid producing ability.

## Description

### [TECHNICAL FIELD]

Provided are a microorganism having enhanced lipoate protein ligase activity, a composition for producing pantothenic acid and/or pantoic acid comprising the microorganism, and a method for producing pantothenic acid and/or pantoic acid comprising culturing the microorganism.

### Cross-reference to Related Application(s)

This disclosure claims the benefit of priority to Korean Patent Application No. 10-2024-0067864, filed on May 24, 2024, and the entire disclosure of said Korean patent application is incorporated herein by reference.

### [BACKGROUND ART]

Pantothenic acid is a substance belonging to the vitamin B complex and is also called vitamin B5, and is one of the commercially important substances variously applied in cosmetics, medicine, human nutrition, animal nutrition, and the like. Pantothenic acid has a structure in which beta-alanine is linked to pantoic acid through an amide bond.

Pantothenic acid or pantoic acid may be produced by chemical synthesis or may be biotechnologically produced by fermenting suitable microorganisms in a suitable medium. An advantage of the biotechnological production method using microorganisms is that the desired stereoisomeric D-form of pantothenic acid or pantoic acid is formed.

Accordingly, development of a microorganism having an advantageous effect in biotechnologically producing pantothenic acid and/or pantoic acid and a technology for producing pantothenic acid and/or pantoic acid with high efficiency using the same is required.

### [Prior Art Documents]

(Patent Document 1) U.S. Patent No. 7,718,205

### [DETAILED DESCRIPTION OF THE INVENTION]

### [TECHNICAL PROBLEM]

One embodiment of the present disclosure provides a microorganism producing pantothenic acid or pantoic acid, in which activity of lipoate protein ligase is enhanced.

Another embodiment of the present disclosure provides a composition for producing pantothenic acid or pantoic acid, comprising the microorganism.

Yet another embodiment of the present disclosure provides a use of the microorganism for producing pantothenic acid or pantoic acid.

Yet another embodiment of the present disclosure provides a use of the microorganism for the preparation of a composition for producing pantothenic acid or pantoic acid.

Yet another embodiment of the present disclosure provides a method for producing pantothenic acid or pantoic acid, comprising culturing the microorganism in a medium.

### [TECHNICAL SOLUTION]

One aspect provides a microorganism producing pantothenic acid or pantoic acid, in which lipoate protein ligase activity is enhanced.

The lipoate protein ligase (or lipoate protein ligase A) may be an enzyme that catalyzes a process of converting and activating ATP and lipoic acid into lipoyl-AMP and transferring the lipoyl to a lipoyl domain of a lipoate-dependent enzyme. The lipoate protein ligase may refer to a lipoate protein ligase protein, a lipoate protein ligase polypeptide, or a protein having lipoate protein ligase activity. The lipoate protein ligase is known in the art and may specifically be an LplA protein encoded by an lplA gene, but is not limited thereto.

The microorganism producing pantothenic acid or pantoic acid, in which lipoate protein ligase activity is enhanced, may be a microorganism into which the lipoate protein ligase or a polynucleotide encoding the same has been introduced. The lipoate protein ligase may be an endogenous protein of the microorganism into which it is introduced, or an exogenous protein.

In one embodiment, the lipoate protein ligase may comprise or consist of an amino acid sequence having at least 70%, 75%, 80%, 85%, 90%, 95%, 96%, 97%, 98%, 99%, 99.5%, 99.7%, or 99.9% homology or identity to the amino acid sequence of SEQ ID NO: 1. In addition, any variant of the lipoate protein ligase that has such homology or identity and exhibits lipoate protein ligase activity, such as one having an amino acid sequence with a deletion, modification, substitution, conservative substitution, or addition of a portion of the sequence, may also be included in the scope of the lipoate protein ligase. For example, such a variant may have a sequence addition or deletion, a naturally occurring mutation, a silent mutation, or a conservative substitution at the N-terminus, C-terminus, and/or within the amino acid sequence, which does not alter lipoate protein ligase activity.

The "conservative substitution" means substituting one amino acid with another amino acid having similar structural and/or chemical properties. Such amino acid substitutions may generally occur based on similarity in polarity, charge, solubility, hydrophobicity, hydrophilicity, and/or amphipathic nature of the residues. Typically, a conservative substitution may have little or no effect on the activity of a protein or polypeptide.

In the present disclosure, the introduction of an exogenous protein or a polynucleotide encoding the same into a host cell (microorganism) may mean the introduction of a protein derived from a cell belonging to a different genus, a cell belonging to a different species, or a different cell of the same species as the host cell, or a polynucleotide encoding the same, into the host cell.

In one embodiment, the lipoate protein ligase and/or the polynucleotide encoding the same may be derived from a microorganism of the genus *Bacillus,* but are not limited thereto.

The lipoate protein ligase and/or the polynucleotide encoding the same derived from a microorganism of the genus *Bacillus* may be derived from a microorganism selected from the group consisting of *Bacillus subtilis, Bacillus amyloliquefaciens, Bacillus methylotrophicus, Bacillus licheniformis, Bacillus velezensis, Bacillus sonorensis,* and *Bacillus valismortis,* but are not limited thereto. In one embodiment, the exogenous lipoate protein ligase and/or the polynucleotide encoding the same may be derived from *Bacillus subtilis.* The sequence of the *Bacillus subtilis-*derived lipoate protein ligase may be obtained from NCBI, which is a known database.

In one embodiment, the *Bacillus subtilis*-derived lipoate protein ligase may have the amino acid sequence of SEQ ID NO: 1, comprise the amino acid sequence of SEQ ID NO: 1, consist of the amino acid sequence of SEQ ID NO: 1, or consist essentially of the amino acid sequence of SEQ ID NO: 1.

The polynucleotide encoding the lipoate protein ligase may refer to a gene encoding the lipoate protein ligase.

The polynucleotide encoding the *Bacillus subtilis-derived* lipoate protein ligase may be derived from *Bacillus subtilis,* but is not limited thereto, and may be prepared based on the amino acid sequence of the *Bacillus subtilis-derived* lipoate protein ligase with reference to a known codon table.

In one embodiment, the polynucleotide encoding the *Bacillus subtilis-derived* lipoate protein ligase may have the nucleic acid sequence of SEQ ID NO: 5, comprise the nucleic acid sequence of SEQ ID NO: 5, consist of the nucleic acid sequence of SEQ ID NO: 5, or consist essentially of the nucleic acid sequence of SEQ ID NO: 5.

In one embodiment, the polynucleotide encoding the lipoate protein ligase may comprise or consist of, a nucleic acid sequence having at least 70%, 75%, 80%, 85%, 90%, 95%, 96%, 97%, 98%, 99%, 99.5%, 99.7%, or 99.9% homology or identity to the nucleic acid sequence of SEQ ID NO: 5.

In one embodiment, the microorganism producing pantothenic acid or pantoic acid, in which lipoate protein ligase activity is enhanced, may be a microorganism into which lipoate protein ligase derived from *Bacillus subtilis* or a polynucleotide encoding the same is introduced.

The lipoate protein ligase may be introduced into a microorganism using a recombinant vector. The recombinant vector may be used as an insertion vector or an expression vector. Expressing the lipoate protein ligase in a microorganism may be performed by culturing a recombinant cell (e.g., a microorganism) comprising a lipoate protein ligase gene, a polynucleotide encoding the lipoate protein ligase, or a recombinant vector comprising the same.

Introduction of the polynucleotide encoding the lipoate protein ligase or the recombinant vector into a microorganism may be performed by a person skilled in the art appropriately selecting a known transformation method. In the present disclosure, the term "transformation" means changing genetic traits of a host cell by introducing a vector comprising a target polynucleotide into the host cell. The transformed polynucleotide may be inserted into and located in the chromosome of the host cell or may be located extrachromosomally. There is no limitation on the form in which the polynucleotide is introduced, as long as it may be introduced into and expressed in the host cell. For example, the polynucleotide may be introduced into the host cell in the form of an expression cassette, which is a gene construct comprising all elements necessary for expression thereof. The expression cassette may typically comprise expression regulatory elements such as a promoter, a transcription termination signal, a ribosome binding site, and/or a translation termination signal, which are operably linked to the polynucleotide. The expression cassette may be in the form of an expression vector capable of self-replication. In addition, the polynucleotide may be introduced into the host cell in its own form and operably linked to sequences necessary for expression in the host cell. As used herein, the term "operably linked" may mean that an expression regulatory element (e.g., a promoter) and a polynucleotide are functionally connected so as to perform transcriptional regulation (e.g., initiation of transcription) of the polynucleotide. Operable linkage may be performed using genetic engineering techniques known in the art.

The method for transforming a host cell with the polynucleotide may be performed by any method for introducing a nucleic acid into a cell (microorganism), and a transformation technique known in the art may be appropriately selected according to the host cell and performed. Examples of the known transformation methods include, but are not limited to, electroporation, calcium phosphate (CaPO₄) precipitation, calcium chloride (CaCl₂) precipitation, microinjection, polyethylene glycol (PEG) precipitation (polyethylene glycol-mediated uptake), DEAE-dextran method, cationic liposome method, lipofection, and lithium acetate-DMSO method.

In the present disclosure, the term "vector" refers to a DNA preparation for delivering a target polynucleotide into a suitable host or host cell. As an example, a vector may comprise a nucleotide sequence of a polynucleotide operably linked to a suitable regulatory sequence so that the target polynucleotide may be expressed in a suitable host. The regulatory sequence may comprise a promoter capable of initiating transcription, any operator sequence for regulating transcription, a sequence encoding a suitable mRNA ribosome binding site, and/or a sequence regulating the termination of transcription and/or translation. A vector, after being transformed into a suitable host cell, may replicate or function independently of the genome of the host cell, or may replicate or function by being integrated into the genome itself.

A vector usable in the present disclosure is not particularly limited as long as it is replicable in a host cell, and may be selected from all commonly used vectors. Examples of commonly used vectors include plasmids, cosmids, viruses, bacteriophages, and the like in a natural or recombinant state. For example, as the vector, pWE15, M13, MBL3, MBL4, IXII, ASHII, APII, t10, t11, Charon4A, and Charon21A, and the like may be used as phage vectors or cosmid vectors, and pBR-based, pUC-based, pBluescriptII-based, pGEM-based, pTZ-based, pCL-based, and pET-based vectors, and the like may be used as plasmid vectors. Specifically, pDZ, pDC, pDCM2, pACYC177, pACYC184, pCL, pECCG117, pUC19, pBR322, pMW118, and pCC1BAC vectors may be exemplified, but is not limited thereto.

The vector may further comprise a selection marker for identifying whether transformation into a host cell has occurred or, furthermore, whether insertion into a chromosome within the host cell has occurred. The selection marker is for selecting cells transformed with a vector or for identifying whether a target polynucleotide has been inserted into a chromosome, and markers that confer selectable phenotypes such as drug resistance, auxotrophy, resistance to cytotoxic agents, or expression of surface proteins may be used. In an environment treated with a selective agent, only cells expressing the selection marker survive or exhibit other phenotypes, so transformed cells may be selected.

In the present disclosure, that a polynucleotide or polypeptide "has, comprises, consists of, or consists essentially of a specific nucleic acid sequence (nucleotide sequence) or amino acid sequence" may mean that the polynucleotide or polypeptide essentially comprises the specific nucleic acid sequence (nucleotide sequence) or amino acid sequence, and may be interpreted as including a "substantially equivalent sequence" in which a variation (deletion, substitution, modification, and/or addition) is applied to the specific nucleic acid sequence (nucleotide sequence) or amino acid sequence within a range that maintains the original function and/or desired function of the polynucleotide or polypeptide (or as not excluding the variation). In one example, that a polynucleotide or polypeptide "has, comprises, consists of, or consists essentially of a specific nucleic acid sequence (nucleotide sequence) or amino acid sequence" may mean that the polynucleotide or polypeptide (i) essentially comprises the specific nucleic acid sequence (nucleotide sequence) or amino acid sequence, or (ii) consists of or essentially comprises a nucleic acid sequence or amino acid sequence having 70% or more, 80% or more, 85% or more, 90% or more, 91% or more, 92% or more, 93% or more, 94% or more, 95% or more, 96% or more, 97% or more, 98% or more, 98% or more, 99% or more, 99.5% or more, or 99.9% or more homology or identity with the specific nucleic acid sequence (nucleotide sequence) or amino acid sequence and maintains the original function and/or desired function. In one example, the desired function may mean a function of increasing (improving) or conferring pantothenic acid and/or pantoic acid production ability of a microorganism.

In the present disclosure, 'homology' or 'identity' refers to a degree of similarity between two given amino acid sequences or nucleotide sequences and may be expressed as a percentage. The terms homology and identity may often be used interchangeably.

Sequence homology or identity of a conserved polynucleotide or polypeptide is determined by standard alignment algorithms, and a default gap penalty established by the program used may be used together. Substantially, homologous or identical sequences generally may hybridize with the entire sequence or a portion thereof under medium or high stringency conditions. It is self-evident that hybridization also includes hybridization with a polynucleotide containing a common codon or a codon in view of codon degeneracy in the polynucleotide.

Whether any two polynucleotide or polypeptide sequences have homology, similarity, or identity may be determined using a known computer algorithm such as the "FASTA" program using default parameters as in, for example, Pearson et al (1988) [Proc. Natl. Acad. Sci. USA 85]: 2444. Alternatively, it may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including the GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego, 1994, and [CARILLO ETA/.] (1988) SIAM J Applied Math 48: 1073)). For example, homology, similarity, or identity may be determined using BLAST of the National Center for Biotechnology Information, or ClustalW.

Homology, similarity, or identity of a polynucleotide or polypeptide may be determined by comparing sequence information using a GAP computer program, such as, for example, Needleman et al. (1970), J Mol Biol. 48:443, as known in, for example, Smith and Waterman, Adv. Appl. Math (1981) 2:482. In summary, the GAP program may be defined as a value obtained by dividing the number of similarly arranged symbols (i.e., nucleotides or amino acids) by the total number of symbols in the shorter of the two sequences. Default parameters for the GAP program may comprise (1) a binary comparison matrix (containing a value of 1 for identity and 0 for non-identity) and a weighted comparison matrix (or EDNAFULL (EMBOSS version of NCBI NUC4.4) substitution matrix) of Gribskov et al (1986) Nucl. Acids Res. 14: 6745, as disclosed by Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap opening penalty 10, gap extension penalty 0.5); and (3) no penalty for terminal gaps.

In the present disclosure, the term "microorganism (or strain)" may include both wild-type microorganisms and microorganisms in which genetic modification has occurred naturally or artificially. The microorganism is a microorganism in which a specific mechanism is enhanced or weakened due to, for example, insertion of an exogenous gene or enhancement or weakening of the activity of an endogenous gene, and may be a microorganism that has undergone a genetic modification for the production of a desired polypeptide, protein, or product (e.g., pantothenic acid and/or pantoic acid).

In the present disclosure, the term "enhancement" of polypeptide (protein) activity means that the activity of the polypeptide is increased compared to its intrinsic activity. The term "enhancement" may be used interchangeably with terms such as activation, up-regulation, overexpression, and increase. Here, activation, enhancement, up-regulation, overexpression, and increase may all include exhibiting activity that was not originally possessed, or exhibiting improved activity compared to intrinsic activity or activity before modification. The term "intrinsic activity" means the activity of a specific polypeptide originally possessed by a parent strain or an unmodified microorganism before a phenotypic change when the phenotype is changed by genetic mutation due to natural or artificial factors. This term may be used interchangeably with "activity before modification." That the activity of a polypeptide is "enhanced," "up-regulated," "overexpressed," or "increased" compared to its intrinsic activity means that it is improved compared to the activity and/or concentration (expression level) of a specific polypeptide originally possessed by the parent strain or the unmodified microorganism before the phenotypic change.

The enhancement may be achieved by introducing an exogenous polypeptide or through enhancement of activity and/or increase in concentration (expression level) of an endogenous polypeptide. Whether the activity of the polypeptide is enhanced may be confirmed from an increase in the degree of activity of the corresponding polypeptide, the expression level, or the amount of a product produced by the activity of the corresponding polypeptide.

For the enhancement of the activity of the polypeptide, various methods well known in the art may be applied, and there is no limitation as long as the activity of the target polypeptide may be enhanced compared to that of the microorganism before modification. Specifically, the enhancement may be performed using genetic engineering and/or protein engineering well known to those of ordinary skill in the art, which are routine methods of molecular biology, but is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the enhancement of the activity of the polypeptide (protein) of the present disclosure may be by
1) an increase in the intracellular copy number of a polynucleotide encoding the polypeptide;
2) replacement of the expression regulatory region of a gene on a chromosome encoding the polypeptide with a sequence having strong activity;
3) modification of a nucleotide sequence encoding a start codon or a 5'-UTR region of a gene transcript encoding the polypeptide;
4) modification of the amino acid sequence of the polypeptide such that the activity of the polypeptide is enhanced;
5) modification of a polynucleotide sequence encoding the polypeptide such that the activity of the polypeptide is enhanced (e.g., modification of the polynucleotide sequence of the polypeptide gene so as to encode a polypeptide modified such that the activity of the polypeptide is enhanced);
6) introduction of an exogenous polypeptide exhibiting the activity of the polypeptide or an exogenous polynucleotide encoding the same;
7) codon optimization of a polynucleotide encoding the polypeptide;
8) selection of an exposed site by analyzing the tertiary structure of the polypeptide, and modification or chemical modification of the selected exposed site;
9) regulation of intracellular localization of the polypeptide; or
10) a combination of two or more selected from the group consisting of the above items 1) to 9), but is not particularly limited thereto.

### More specifically,

The increase in the intracellular copy number of the polynucleotide encoding the polypeptide as described in the above item 1) may be achieved by introduction into a host cell of a vector which may replicate and function independently of the host and to which the polynucleotide encoding the corresponding polypeptide is operably linked. Alternatively, it may be achieved by the introduction of 1 copy or 2 or more copies of the polynucleotide encoding the corresponding polypeptide into the chromosome within the host cell. The introduction into the chromosome may be performed by introducing into the host cell a vector capable of inserting the polynucleotide into the chromosome within the host cell, but is not limited thereto. The vector is as described above.

The replacement of the expression regulatory region (or expression regulatory sequence) of a gene on a chromosome encoding the polypeptide with a sequence having strong activity as described in the above item 2) may be, for example, the generation of a sequence variation by deletion, insertion, non-conservative or conservative substitution, or a combination thereof so as to further enhance the activity of the expression regulatory region, or replacement with a sequence having stronger activity. The expression regulatory region, without being particularly limited thereto, may include a promoter, an operator sequence, a sequence encoding a ribosome binding site, and a sequence regulating the termination of transcription and translation. As an example, it may be the replacement of the original promoter with a strong promoter, but is not limited thereto.

Examples of known strong promoters include, but are not limited to, CJ1 to CJ7 promoters (U.S. Patent No. 7,662,943 B2), lac promoter, trp promoter, trc promoter, tac promoter, lambda phage PR promoter, PL promoter, tet promoter, gapA promoter, SPL7 promoter, SPL13(sm3) promoter (U.S. Patent No. 10,584,338 B2), O2 promoter (U.S. Patent No. 10,273,491 B2), tkt promoter, and yccA promoter.

The modification of the nucleotide sequence encoding the start codon or 5'-UTR region of the gene transcript encoding the polypeptide as described in the above item 3) may be, for example, substitution with a nucleotide sequence encoding another start codon having a higher polypeptide expression rate compared to the endogenous start codon, but is not limited thereto.

The modification of the amino acid sequence or polynucleotide sequence as described in the above items 4) and 5) may be, but is not limited to, the generation of a sequence variation by deletion, insertion, non-conservative or conservative substitution, or a combination thereof in the amino acid sequence of the polypeptide or the polynucleotide sequence encoding the polypeptide so as to enhance the activity of the polypeptide, or replacement with an amino acid sequence or polynucleotide sequence improved such that the activity is stronger or an amino acid sequence or polynucleotide sequence improved such that the activity is increased. The replacement may be specifically performed by inserting the polynucleotide into the chromosome by homologous recombination, but is not limited thereto. The vector used at this time may further comprise a selection marker for identifying whether insertion into the chromosome has occurred.

The introduction of the exogenous polynucleotide exhibiting the activity of the polypeptide as described in the above item 6) may be the introduction into a host cell of an exogenous polynucleotide encoding a polypeptide exhibiting the same as or similar to the activity of the polypeptide. The exogenous polynucleotide is not limited in its origin or sequence as long as it exhibits the same as or similar to the activity of the polypeptide. The method used for the introduction may be performed by a person skilled in the art appropriately selecting a known transformation method, and by expressing the introduced polynucleotide in the host cell, a polypeptide is produced, and its activity may be increased.

The codon optimization of the polynucleotide encoding the polypeptide as described in the above item 7) may be an optimization of an endogenous polynucleotide such that transcription or translation thereof is increased in a host cell, or an optimization of an exogenous polynucleotide such that transcription and translation thereof are optimized in a host cell.

The selection of an exposed site by analyzing the tertiary structure of the polypeptide, and modification or chemical modification of the selected exposed site as described in the above item 8) may be, for example, performed by comparing sequence information of a polypeptide to be analyzed with a database storing sequence information of known proteins to determine a template protein candidate according to the degree of sequence similarity, identifying the structure based thereon, and selecting and then modifying or chemically modifying the exposed site to be modified or chemically modified.

The regulation of intracellular localization of the polypeptide as described in the above item 9) may comprise targeting the polypeptide to a specific intracellular organelle or a specific intracellular space. For example, the regulation may comprise targeting the polypeptide to the periplasm or cytoplasm through the addition or removal of a leader sequence that functions in targeting the polypeptide, but is not limited thereto.

Such enhancement of polypeptide activity may be achieved by an increase in the activity or the concentration expression level of the corresponding polypeptide relative to the activity or concentration of the polypeptide expressed in a wild-type or unmodified microbial strain, or by an increase in the amount of a product produced from the corresponding polypeptide, but is not limited thereto.

In the present disclosure, the term "weakening" of activity of a polypeptide (protein) is a concept that includes both a decrease in activity compared to intrinsic activity and the absence of activity. The term "weakening" may be used interchangeably with terms such as inactivation, deficiency, down-regulation, decrease, reduction, and attenuation.

The weakening may also include a case where the activity of the polypeptide itself is reduced or eliminated compared to the activity of the polypeptide possessed by the original microorganism due to a mutation of a polynucleotide encoding the polypeptide, a case where the overall degree of polypeptide activity and/or concentration (expression level) in the cell is lower than that of a natural strain due to inhibition of expression of a gene of a polynucleotide encoding the same or inhibition of translation into a polypeptide, a case where expression of the polynucleotide is not performed at all, and/or a case where there is no activity of the polypeptide even if the polynucleotide is expressed. The term "intrinsic activity" refers to the activity of a specific polypeptide originally possessed by a parent strain, a wild-type, or an unmodified microorganism before a phenotypic change when the trait is changed by a genetic mutation due to natural or artificial factors. This term may be used interchangeably with "activity before modification." That the activity of a polypeptide is "inactivated, deficient, decreased, down-regulated, reduced, or weakened" compared to its intrinsic activity means that it has become lower than the activity of a specific polypeptide originally possessed by the parent strain or the unmodified microorganism before the phenotypic change.

Such weakening of the activity of the polypeptide may be performed by any method known in the art, but is not limited thereto, and may be achieved by applying various methods well known in the art (e.g., Nakashima N et al., Bacterial cellular engineering by genome editing and gene silencing. Int J Mol Sci. 2014;15(2):2773-2793, Sambrook et al. Molecular Cloning 2012, etc.).

Specifically, the weakening of the activity of the polypeptide (protein) may be
1) deletion of all or part of a gene encoding the polypeptide;
2) modification of an expression regulatory region (or expression regulatory sequence) such that the expression of a gene encoding the polypeptide is decreased;
3) modification of an amino acid sequence constituting the polypeptide such that the activity of the polypeptide is eliminated or weakened (e.g., deletion/substitution/addition of one or more amino acids in the amino acid sequence);
4) modification of a gene sequence encoding the polypeptide such that the activity of the polypeptide is eliminated or weakened (e.g., deletion/substitution/addition of one or more nucleic acid bases in the nucleic acid sequence of the gene encoding the polypeptide so as to encode a polypeptide modified such that the activity of the polypeptide is eliminated or weakened);
5) modification of a nucleotide sequence encoding a start codon or a 5'-UTR region of a gene transcript encoding the polypeptide;
6) introduction of an antisense oligonucleotide (e.g., antisense RNA) that complementarily binds to the transcript of the gene encoding the polypeptide;
7) addition of a sequence complementary to a Shine-Dalgarno sequence to upstream of the Shine-Dalgarno sequence of a gene encoding the polypeptide in order to form a secondary structure to which a ribosome may not attach;
8) addition of a promoter that is transcribed in the opposite direction to the 3' end of an open reading frame (ORF) of a gene sequence encoding the polypeptide (Reverse transcription engineering (RTE));
9) regulation of intracellular localization of the polypeptide; or
10) a combination of two or more selected from the group consisting of the above items 1) through 9), but is not particularly limited thereto.

For example, the deletion of part or all of the gene encoding the polypeptide as described in the above item 1) may be removal of the entire polynucleotide encoding the endogenous target polypeptide in a chromosome, replacement with a polynucleotide in which some nucleotides are deleted, or replacement with a marker gene.

In addition, the modification of the expression regulatory region (or expression regulatory sequence) as described in the above item 2) may be the generation of a variation in the expression regulatory region (or expression regulatory sequence) by deletion, insertion, non-conservative or conservative substitution, or a combination thereof, or a replacement with a sequence having weaker activity. The expression regulatory region may include, but is not limited to, a promoter, an operator sequence, a sequence encoding a ribosome binding site, and a sequence regulating the termination of transcription and translation.

In addition, the modification of the nucleotide sequence encoding the start codon or 5'-UTR region of the gene transcript encoding the polypeptide as described in the above item 3) may be, for example, substitution with a nucleotide sequence encoding another start codon having a lower polypeptide expression rate compared to the endogenous start codon, but is not limited thereto.

In addition, the modification of the amino acid sequence or polynucleotide sequence as described in the above items 4) and 5) may be, but is not limited to, the generation of a variation in the sequence by deletion, insertion, non-conservative or conservative substitution, or a combination thereof in the amino acid sequence of the polypeptide or the polynucleotide sequence encoding the polypeptide so as to weaken the activity of the polypeptide, or replacement with an amino acid sequence or polynucleotide sequence improved to have weaker activity or an amino acid sequence or polynucleotide sequence improved to have no activity. For example, by introducing a mutation into a polynucleotide sequence to form a stop codon, expression of a gene may be inhibited or weakened, but is not limited thereto. The "stop codon" is a codon that does not designate an amino acid among codons on mRNA and acts as a signal that the protein synthesis process has ended, and generally, three types of UAA, UAG, and UGA may be used as stop codons.

The introduction of an antisense oligonucleotide (e.g., antisense RNA) that complementarily binds to the transcript of the gene encoding the polypeptide as described in the above item 6) may refer to, for example, the literature [Weintraub, H. et al., Antisense-RNA as a molecular tool for genetic analysis, Reviews - Trends in Genetics, Vol. 1(1) 1986].

The addition of a sequence complementary to a Shine-Dalgarno sequence upstream of the Shine-Dalgarno sequence of a gene encoding the polypeptide in order to form a secondary structure to which a ribosome is unable to attach as described in the above item 7) may render mRNA translation impossible or reduce the rate thereof.

The addition of a promoter, which is transcribed in the opposite direction, to the 3' end of an open reading frame (ORF) of a gene sequence encoding the polypeptide (Reverse transcription engineering (RTE)) as described in the above item 8) may weaken the activity by generating an antisense nucleotide complementary to a transcript of the gene encoding the polypeptide.

The regulation of the intracellular localization of the polypeptide as described in the above item 9) may involve targeting the polypeptide to a specific intracellular organelle or a specific intracellular space within a cell. For example, the regulation may involve targeting to the periplasm or cytoplasm through the addition or removal of a leader sequence that functions in the targeting of the polypeptide, but is not limited thereto.

Such weakening of polypeptide activity may be that the activity or concentration expression level of the corresponding polypeptide is weakened based on the activity or concentration of the polypeptide expressed in a wild-type or unmodified microbial strain, or that the amount of a product produced from the corresponding polypeptide is increased, but is not limited thereto.

Another aspect provides a microorganism of the genus *Corynebacterium* producing pantothenic acid or pantoic acid, in which lipoate protein ligase activity is enhanced.

The microorganism (or strain, recombinant cell) of the present disclosure may be a microorganism having pantothenic acid and/or pantoic acid production ability (or production amount), or having improved (or increased) pantothenic acid and/or pantoic acid production ability.

The microorganism producing pantothenic acid or pantoic acid, in which lipoate protein ligase activity is enhanced, of the present disclosure may be a microorganism into which the lipoate protein ligase or a polynucleotide encoding the same has been introduced, wherein the microorganism is a microorganism that does not naturally have pantothenic acid and/or pantoic acid production ability, or a microorganism that has pantothenic acid and/or pantoic acid production ability, but is not limited thereto. In one embodiment, in the microorganism, the polynucleotide encoding the lipoate protein ligase may be operably linked to a strong promoter. In one embodiment, the lipoate protein ligase may be derived from *Bacillus subtilis*. The microorganism in which the activity of lipoate protein ligase derived from Bacillus subtilis is enhanced may be one in which the pantothenic acid and/or pantoic acid production ability is improved.

The fact that the microorganism with enhanced lipoate protein ligase activity has improved pantothenic acid and/or pantoic acid production ability or possesses pantothenic acid and/or pantoic acid production ability may mean that the microorganism has improved pantothenic acid and/or pantoic acid production ability compared to an unmodified microorganism, a cell before recombination, a parent strain, or a wild-type microorganism, or that pantothenic acid and/or pantoic acid production ability is conferred thereto, unlike an unmodified microorganism, a cell before recombination, a parent strain, or a wild-type microorganism that lacks pantothenic acid and/or pantoic acid production ability.

A microorganism having enhanced lipoate protein ligase activity according to one embodiment may be one in which pantothenic acid and/or pantoic acid production ability is improved as compared to a microorganism before enhancement, that is, an unmodified microorganism of the same species. In the present disclosure, "unmodified microorganism" does not exclude a strain comprising a mutation that may naturally occur in a microorganism, and may mean a wild-type strain or a natural strain itself, or a strain before a phenotype is changed by a genetic mutation due to natural or artificial factors. For example, the unmodified microorganism may mean a strain before the activity of lipoate protein ligase is enhanced according to one embodiment. The "unmodified microorganism" may be used interchangeably with "pre-modification strain," "pre-modification microorganism," "non-mutant strain," "unmodified strain," "non-mutant microorganism," or "reference microorganism." That lipoate protein ligase activity is enhanced is as described above.

In one embodiment, the microorganism producing pantothenic acid or pantoic acid, in which lipoate protein ligase activity is enhanced, may be a microorganism in which glycine cleavage system activity is further enhanced.

The microorganism in which glycine cleavage system activity is enhanced may be a microorganism into which an exogenous glycine cleavage system or a polynucleotide encoding the same is introduced.

The glycine cleavage system may comprise a complex of glycine cleavage enzymes, or one or more types of glycine cleavage enzymes.

The glycine cleavage enzyme may be any one enzyme selected from the group consisting of glycine dehydrogenase, aminomethyltransferase, glycine decarboxylase, and dihydrolipoyl dehydrogenase.

The glycine cleavage system may comprise one or more subunits selected from the group consisting of a GcvP polypeptide, a GcvT polypeptide, and a GcvH polypeptide. In one embodiment, the glycine cleavage system may comprise a GcvP polypeptide, a GcvT polypeptide, and a GcvH polypeptide.

The exogenous glycine cleavage system may be such that each of one or more subunits selected from the group consisting of GcvP, GcvT, and GcvH polypeptides is derived from different exogenous microorganisms, or all of the one or more subunits are derived from the same exogenous microorganism.

The exogenous glycine cleavage system and/or the polynucleotide encoding the same may be derived from a microorganism of the genus *Mycobacterium,* but are not limited thereto.

The exogenous glycine cleavage system and/or the polynucleotide encoding the same may be derived from one or more microorganisms of the genus *Mycobacterium* selected from the group consisting of *Mycobacterium abscessus, Mycobacterium africanum, Mycobacterium asiaticum, Mycobacterium bovis, Mycobacterium chelonae, Mycobacterium fortuitum, Mycobacterium gordonae, Mycobacterium haemophilum, Mycobacterium intracellulare, Mycobacterium kansasii, Mycobacterium lentiflavum, Mycobacterium malmoense, Mycobacterium marinum, Mycobacterium microti, Mycobacterium phlei, Mycobacterium scrofulaceum, Mycobacterium smegmatis, Mycobacterium triplex, Mycobacterium uvium,* and *Mycobacterium xenopi,* but are not limited thereto. In one embodiment, the exogenous glycine cleavage system and/or the polynucleotide encoding the same may be derived from *Mycobacterium smegmatis.* The sequences of the GcvP, GcvT, or GcvH polypeptides derived from *Mycobacterium smegmatis* may be obtained from NCBI, which is a known database, as an example.

In one embodiment, the *Mycobacterium smegmatis-derived* GcvP polypeptide may have, comprise, consist of, or consist essentially of the amino acid sequence of SEQ ID NO: 25.

In one embodiment, the GcvP polypeptide may comprise or consist of an amino acid sequence having at least 70%, 75%, 80%, 85%, 90%, 95%, 96%, 97%, 98%, 99%, 99.5%, 99.7%, or 99.9% homology or identity to the amino acid sequence of SEQ ID NO: 25. In addition, any variant of the GcvP polypeptide that has such homology or identity and exhibits glycine dehydrogenase activity, such as one having an amino acid sequence with a deletion, modification, substitution, conservative substitution, or addition of a portion of the sequence, may also be included in the GcvP polypeptide. For example, such a variant may have a sequence addition or deletion, a naturally occurring mutation, a silent mutation, or a conservative substitution at the N-terminus, C-terminus, and/or within the amino acid sequence, which does not alter the glycine dehydrogenase activity.

The polynucleotide encoding the GcvP polypeptide derived from *Mycobacterium smegmatis* may be derived from *Mycobacterium smegmatis*, but is not limited thereto, and may be prepared based on the amino acid sequence of the GcvP polypeptide by referring to a known codon table. In one embodiment, the polynucleotide encoding the GcvP polypeptide derived from *Mycobacterium smegmatis* may have the nucleic acid sequence of SEQ ID NO: 4 or SEQ ID NO: 28, comprise the nucleic acid sequence of SEQ ID NO: 4 or SEQ ID NO: 28, consist of the nucleic acid sequence of SEQ ID NO: 4 or SEQ ID NO: 28, or consist essentially of the nucleic acid sequence of SEQ ID NO: 4 or SEQ ID NO: 28.

In one embodiment, the polynucleotide encoding the GcvP polypeptide may comprise or consist of a nucleic acid sequence having at least 70%, 75%, 80%, 85%, 90%, 95%, 96%, 97%, 98%, 99%, 99.5%, 99.7%, or 99.9% homology or identity to the nucleic acid sequence of SEQ ID NO: 4 or SEQ ID NO: 28.

In one embodiment, the GcvT polypeptide derived from *Mycobacterium smegmatis* may have, comprise, consist of, or consist essentially of the amino acid sequence of SEQ ID NO: 26.

In one embodiment, the GcvT polypeptide may comprise or consist of an amino acid sequence having at least 70%, 75%, 80%, 85%, 90%, 95%, 96%, 97%, 98%, 99%, 99.5%, 99.7%, or 99.9% homology or identity to the amino acid sequence of SEQ ID NO: 26. In addition, any variant of the GcvP polypeptide that has such homology or identity and exhibits glycine dehydrogenase activity, such as one having an amino acid sequence with a deletion, modification, substitution, conservative substitution, or addition of a portion of the sequence, may also be included in the GcvP polypeptide. For example, such a variant may have a sequence addition or deletion, a naturally occurring mutation, a silent mutation, or a conservative substitution at the N-terminus, C-terminus, and/or within the amino acid sequence, which does not alter the glycine dehydrogenase activity.

The polynucleotide encoding the GcvT polypeptide derived from *Mycobacterium smegmatis* may be derived from *Mycobacterium smegmatis*, but is not limited thereto, and may be prepared based on the amino acid sequence of the GcvT polypeptide and with reference to a known codon table. In one embodiment, the polynucleotide encoding the GcvT polypeptide derived from *Mycobacterium smegmatis* may have, comprise, consist of, or consist essentially of the nucleic acid sequence of SEQ ID NO: 5 or SEQ ID NO: 29.

In one embodiment, the polynucleotide encoding the GcvT polypeptide may comprise or consist of, a nucleic acid sequence having at least 70%, 75%, 80%, 85%, 90%, 95%, 96%, 97%, 98%, 99%, 99.5%, 99.7%, or 99.9% homology or identity to the nucleic acid sequence of SEQ ID NO: 5 or SEQ ID NO: 29.

In one embodiment, the GcvH polypeptide derived from *Mycobacterium smegmatis* may have, comprise, consist of, or consist essentially of the amino acid sequence of SEQ ID NO: 27.

In one embodiment, the GcvH polypeptide may comprise or consist of an amino acid sequence having at least 70%, 75%, 80%, 85%, 90%, 95%, 96%, 97%, 98%, 99%, 99.5%, 99.7%, or 99.9% homology or identity to the amino acid sequence of SEQ ID NO: 27. In addition, any variant of the GcvP polypeptide that has such homology or identity and exhibits glycine dehydrogenase activity, such as one having an amino acid sequence with a deletion, modification, substitution, conservative substitution, or addition of a portion of the sequence, may also be included in the GcvP polypeptide. For example, such a variant may have a sequence addition or deletion, a naturally occurring mutation, a silent mutation, or a conservative substitution at the N-terminus, C-terminus, and/or within the amino acid sequence, which does not alter the glycine dehydrogenase activity.

The polynucleotide encoding the GcvH polypeptide derived from *Mycobacterium smegmatis* may be derived from *Mycobacterium smegmatis*, but is not limited thereto, and may be prepared based on the amino acid sequence of the GcvH polypeptide by reference to a known codon table. In one embodiment, the polynucleotide encoding the GcvH polypeptide derived from *Mycobacterium smegmatis* may have the nucleic acid sequence of SEQ ID NO: 6 or SEQ ID NO: 30, comprise the nucleic acid sequence of SEQ ID NO: 6 or SEQ ID NO: 30, consist of the nucleic acid sequence of SEQ ID NO: 6 or SEQ ID NO: 30, or consist essentially of the nucleic acid sequence of SEQ ID NO: 6 or SEQ ID NO: 30.

In one embodiment, the polynucleotide encoding the GcvH polypeptide may comprise or consist of a nucleic acid sequence having at least 70%, 75%, 80%, 85%, 90%, 95%, 96%, 97%, 98%, 99%, 99.5%, 99.7%, or 99.9% homology or identity to the nucleic acid sequence of SEQ ID NO: 6 or SEQ ID NO: 30.

The microorganism may additionally comprise a mutation that increases the production of pantothenic acid and/or pantoic acid, and the location of the mutation and/or the type of gene and/or protein targeted by the mutation may be included without limitation, provided that the mutation increases the production of pantothenic acid and/or pantoic acid. The recombinant cell is not limited to any particular type, provided that the cell is capable of being transformed.

The microorganism may be a microorganism of the genus *Corynebacterium*. The microorganism of the genus *Corynebacterium* may be a microorganism selected from the group consisting of *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* and Corynebacterium flavescens, but is not limited thereto.

The microorganism may be a microorganism of the genus *Bacillus.* The microorganism of the genus *Bacillus* may be a microorganism selected from the group consisting of *Bacillus subtilis, Bacillus amyloliquefaciens, Bacillus methylotrophicus, Bacillus licheniformis, Bacillus velezensis, Bacillus sonorensis,* and *Bacillus valismortis,* but is not limited thereto.

The microorganism may be a microorganism of the genus *Escherichia.* The microorganism of the genus *Escherichia* may be *Escherichia coli,* but is not limited thereto.

In one embodiment, the microorganism with enhanced pantothenic acid and/or pantoic acid production ability may have pantothenic acid and/or pantoic acid production ability newly imparted, or increased by about 10% or more, about 20% or more, about 24%, about 25%, about 30% or more, about 40% or more, about 50% or more, about 60% or more, about 70% or more, about 80% or more, about 90% or more, about 100% or more, about 150% or more, about 200% or more, about 250% or more, about 300% or more, about 400% or more, about 500% or more, about 600% or more, about 700% or more, about 800% or more, about 900% or more, about 1,000% or more, about 1,500% or more, about 2,000% or more, about 2,500% or more, or about 3,000% or more compared to a parent strain before mutation, an unmodified microorganism, or a microorganism into which lipoate protein ligase derived from another microorganism or a polynucleotide encoding the same is introduced, but is not limited thereto.

In another embodiment, the microorganism with enhanced pantothenic acid and/or pantoic acid production ability may have pantothenic acid and/or pantoic acid production ability of about 1.1 times or more, about 1.2 times or more, about 1.24 times or more, about 1.3 times or more, about 1.4 times or more, about 1.5 times or more, about 1.6 times or more, about 1.7 times or more, about 1.8 times or more, about 1.9 times or more, about 2 times or more, about 2.5 times or more, about 3 times or more, about 4 times or more, about 5 times or more, about 6 times or more, about 7 times or more, about 8 times or more, about 9 times or more, about 10 times or more, about 15 times or more, about 20 times or more, about 25 times or more, or about 30 times or more (the upper limit is not particularly limited, for example, it may be about 1,000 times or less) compared to a parent strain before mutation, an unmodified microorganism, or a microorganism into which lipoate protein ligase derived from another microorganism or a polynucleotide encoding the same is introduced, but is not limited thereto.

In another embodiment, the microorganism with enhanced pantothenic acid and/or pantoic acid production ability may have pantothenic acid and/or pantoic acid production ability of about 0.1 g/L or more, about 0.2 g/L or more, about 0.3 g/L or more, about 0.4 g/L or more, about 0.5 g/L or more, about 0.6 g/L or more, about 0.7 g/L or more, about 0.8 g/L or more, about 0.9 g/L or more, about 1 g/L or more, about 1.1 g/L or more, about 1.2 g/L or more, about 1.3 g/L or more, about 1.4 g/L or more, about 1.5 g/L or more, about 1.6 g/L or more, about 1.7 g/L or more, about 1.8 g/L or more, about 1.9 g/L or more, about 2.0 g/L or more, about 2.5 g/L or more, about 3 g/L or more, about 3.5 g/L or more, about 4 g/L or more, about 4.5 g/L or more, about 5 g/L or more, about 5.5 g/L or more, about 6 g/L or more, about 7 g/L or more, about 8 g/L or more, about 9 g/L or more, about 10 g/L or more, about 15 g/L or more, about 20 g/L or more, about 25 g/L or more, or about 30 g/L or more (the upper limit is not particularly limited, for example, it may be about 100 g/L or less) compared to a parent strain before mutation, an unmodified microorganism, or a microorganism into which a lipoate protein ligase derived from another microorganism or a polynucleotide encoding the same is introduced, but is not limited thereto.

The term "about" refers to a range encompassing ±0.5, ±0.4, ±0.3, ±0.2, ±0.1, and the like, and includes all values within a range equivalent or similar to the numerical value following the term "about," but is not limited thereto.

Another aspect provides a composition for producing pantothenic acid and/or pantoic acid comprising the microorganism, a medium in which the microorganism is cultured, or a combination thereof.

The composition may further comprise any suitable excipient commonly used in a composition for producing pantothenic acid and/or pantoic acid, and such an excipient may be, for example, a preservative, a wetting agent, a dispersing agent, a suspending agent, a buffer, a stabilizer, or an isotonic agent, but is not limited thereto.

Another aspect provides the use of the microorganism for the production of pantothenic acid and/or pantoic acid.

Another aspect provides the use of the microorganism for the preparation of a composition for producing pantothenic acid and/or pantoic acid.

Another aspect provides a method for producing pantothenic acid and/or pantoic acid, comprising culturing, in a medium, a microorganism of the present disclosure that produces pantothenic acid or pantoic acid, in which lipoate protein ligase activity is enhanced. The microorganism is as described above.

The method for producing pantothenic acid and/or pantoic acid may include culturing the microorganism in a medium.

In the present disclosure, "culturing" means growing the microorganism under appropriately controlled environmental conditions. The culturing process of the present disclosure may be performed according to a suitable medium and culture conditions known in the art. This culturing process may be easily adjusted and used by a person skilled in the art according to the selected strain. Specifically, the culturing may be batch, continuous, and/or fed-batch culture, but is not limited thereto.

In the present disclosure, the term "medium" refers to a substance in which nutrients necessary for culturing the microorganism are mixed as main components, and which supplies nutrients and growth factors, including water, which is essential for survival and growth. Specifically, any medium may be used as the medium and other culture conditions for culturing the microorganism of the present disclosure, provided that it is a medium used for culturing common microorganisms, without particular limitation. However, the microorganism of the present disclosure may be cultured while adjusting the temperature, pH, and the like under aerobic conditions in a common medium containing a suitable carbon source, nitrogen source, phosphate source, inorganic compounds, amino acids, and/or vitamins.

Specifically, a culture medium for the microorganism of the present disclosure, for example, a strain of the genus *Corynebacterium*, may be found in the literature ["Manual of Methods for General Bacteriology" by the American Society for Bacteriology (Washington D.Corynebacterium., USA, 1981)].

In the present disclosure, the carbon source may include carbohydrates such as glucose, saccharose, lactose, fructose, sucrose, and maltose; sugar alcohols such as mannitol and sorbitol; organic acids such as pyruvic acid, lactic acid, and citric acid; and amino acids such as glutamic acid, methionine, and lysine. In addition, natural organic nutrient sources such as starch hydrolysate, molasses (e.g., blackstrap molasses), rice bran, cassava, sugarcane residue, and corn steep liquor may be used; specifically, carbohydrates such as glucose and sterilized pre-treated molasses (i.e., molasses converted to reducing sugar) may be used; and various other carbon sources in appropriate amounts may be used without limitation. These carbon sources may be used alone or in combination of two or more types, but the present disclosure is not limited thereto.

As the nitrogen source, inorganic nitrogen sources such as ammonia, ammonium sulfate, ammonium chloride, ammonium acetate, ammonium phosphate, ammonium carbonate, and ammonium nitrate, amino acids such as glutamic acid, methionine, and glutamine, organic nitrogen sources such as peptone, NZ-amine, meat extract, yeast extract, malt extract, corn steep liquor, casein hydrolysate, fish or a degradation product thereof, and defatted soybean cake or a degradation product thereof may be used. These nitrogen sources may be used alone or in combination of two or more, but are not limited thereto.

The phosphate source may comprise monopotassium phosphate, dipotassium phosphate, or a corresponding sodium-containing salt. The inorganic compound may be selected from the group consisting of sodium chloride, calcium chloride, iron chloride, magnesium sulfate, ferrous sulfate, manganese sulfate, calcium carbonate, and the like, and may additionally comprise amino acids, vitamins, and/or suitable precursors. These components or precursors may be added to the medium in a batch or continuous manner. However, it is not limited thereto.

In addition, during the culture of the microorganism of the present disclosure, a compound such as ammonium hydroxide, potassium hydroxide, ammonia, phosphoric acid, and sulfuric acid may be added to the medium in an appropriate manner to adjust the pH of the medium. In addition, during culture, an antifoaming agent such as fatty acid polyglycol ester may be used to inhibit foam generation. In addition, to maintain an aerobic condition of the medium, oxygen or an oxygen-containing gas may be injected into the medium, or to maintain anaerobic and micro-aerobic conditions, the medium may be maintained without gas injection or by injecting nitrogen, hydrogen, or carbon dioxide gas, but the present disclosure is not limited thereto.

In the culture of the present disclosure, the culture temperature may be maintained at 20 to 45°C, specifically 25 to 40°C, and culture may be performed for about 10 to 160 hours, but is not limited thereto.

Pantothenic acid and/or pantoic acid produced by the culture of the present disclosure may be secreted into the medium or remain in the cells.

The method for producing pantothenic acid and/or pantoic acid of the present disclosure may further include, for example, in any order, a step of preparing the microorganism of the present disclosure, a step of preparing a medium for culturing the microorganism, or a combination thereof, before the culturing step.

The method for producing pantothenic acid and/or pantoic acid of the present disclosure may further include a step of recovering pantothenic acid and/or pantoic acid from the medium according to the culture (the medium in which the culture was performed) or the microorganism (e.g., a strain of the genus *Corynebacterium*). The recovering step may be further included after the culturing step.

The recovery may involve collecting the target pantothenic acid and/or pantoic acid using a suitable method known in the art according to the culture method of the microorganism of the present disclosure, for example, batch, continuous, or fed-batch culture. For example, centrifugation, filtration, treatment with a crystallization protein precipitant (salting-out method), extraction, ultrasonic disruption, ultrafiltration, dialysis, various types of chromatography such as molecular sieve chromatography (gel filtration), adsorption chromatography, ion-exchange chromatography, affinity chromatography, and the like, HPLC, or a combination of these methods may be used, and the target pantothenic acid and/or pantoic acid may be recovered from the medium or the microorganism using a suitable method known in the art.

In addition, the method for producing pantothenic acid and/or pantoic acid of the present disclosure may additionally include a purification step. The purification may be performed using a suitable method known in the art. In one embodiment, when the method for producing pantothenic acid and/or pantoic acid of the present disclosure includes both a recovery step and a purification step, the recovery step and the purification step may be performed continuously or discontinuously regardless of the order, or may be performed simultaneously or integrated into one step, but is not limited thereto.

### [ADVANTAGEOUS EFFECTS]

When culturing a microorganism of the present disclosure having enhanced lipoate protein ligase activity, pantothenic acid and/or pantoic acid may be produced at high yield. Accordingly, effects of production convenience and manufacturing cost reduction may be expected from an industrial perspective.

### [MODE FOR INVENTION]

Hereinafter, the present disclosure will be described in more detail with reference to examples. However, these examples are for illustrative purposes to explain the present disclosure, and the scope of the present disclosure is not limited by these examples, as will be apparent to a person of ordinary skill in the art to which the present disclosure pertains.

### Example 1. Preparation of Microorganism of the Genus Corynebacterium Producing Pantothenic Acid

### (1) Production of Pantothenic Acid-Producing Microorganism

A *Corynebacterium glutamicum* strain having increased pantothenic acid production ability, into which an *Escherichia coli*-derived 3-methyl-2-oxobutanoate hydroxymethyltransferase G116A variant (PanB(G116A), US Patent Publication US 2024-0167065 A1) was introduced, was constructed.

First, a vector for deleting panB present in a parent strain was constructed. Using genomic DNA of *Corynebacterium glutamicum* ATCC13032 as a template, PCR was performed using primers of SEQ ID NOs: 30 and 31 and SEQ ID NOs: 32 and 33. PCR was performed under conditions of repeating 25 cycles of denaturation at 95°C for 30 seconds, annealing at 55°C for 30 seconds, and polymerization at 72°C for 1 minute. As a result, a 1000 bp gene fragment of the upstream region of the panB gene and a 1000 bp gene fragment of the downstream region of the panB gene were obtained, respectively. Each amplification product was purified using a PCR Purification kit from QIAGEN and used as an insert DNA fragment for vector construction. After the pDC24 (SEQ ID NO: 34) vector was treated with the restriction enzyme SmaI and subjected to heat treatment at 65°C for 20 minutes, the vector and the DNA fragments (the 1000 bp gene fragment of the upstream region of the panB gene and the 1000 bp gene fragment of the downstream region of the panB gene) were cloned at a molar ratio of 2:1:1 using an Infusion Cloning Kit from TaKaRa according to the provided manual, thereby constructing a vector pDC24_ΔpanB for deleting the panB gene on the chromosome.

Then, using genomic DNA of the *Escherichia coli* ATCC 47076 strain as a template, PCR was performed using primers of SEQ ID NOs: 35 and 36 and SEQ ID NOs: 37 and 38. The PCR was performed under conditions of repeating 25 cycles of denaturation at 95°C for 30 seconds, annealing at 55°C for 30 seconds, and polymerization at 72°C for 1 minute. As a result, gene fragments of 388 bp at the upstream region and 490 bp at the downstream region of the panB gene were obtained, respectively; each amplification product was purified using a PCR Purification kit from QIAGEN and used as an insert DNA fragment for vector construction. After the pDC24_ΔpanB vector was treated with the restriction enzyme SmaI and subjected to heat treatment at 65°C for 20 minutes, the vector and the obtained DNA fragments were combined to have a molar ratio of 1:1:1 and were cloned using an Infusion Cloning Kit from TaKaRa according to the provided manual, thereby constructing a vector pDC24_ΔpanB::panB(EC, G116A) for introducing the *Escherichia coli*-derived panB (G116A) gene into the chromosome. The constructed vector pDC24_ΔpanB::panB(EC, G116A) was transformed into *Corynebacterium glutamicum* ATCC 13032 through electroporation, and a strain ATCC 13032 ΔpanB::panB(EC, G116A), in which endogenous panB on the chromosome was replaced with the *Escherichia coli*-derived panB(G116A), was obtained through a double crossover process. Whether *Escherichia* coli-derived panB (G116A) was properly substituted was confirmed using a MASA (Mutant Allele Specific Amplification) PCR technique (Takeda et al., Hum. Mutation, 2, 112-117 (1993)) using the following primer combinations. Specifically, primary determination was made by selecting strains amplified using the primer combinations corresponding to *Escherichia coli* panB (SEQ ID NOs: 39 and 40, and SEQ ID NOs: 41 and 42), and the panB sequence of the selected strain was secondarily confirmed by analysis using the primer combination of SEQ ID NO: 39 and SEQ ID NO: 43.

The primer sequences used in the above Example are as shown in Table 1 below.

**[Table 1]**

| SEQ ID NO | Sequence |
|---|---|
| 30 | TGAATTCGAGCTCGGTACCCGAAATAGCGCTTGATGAATC |
| 31 | GGTTGCTACCTGCACCCGGGGGGCATGAGTATAGATGTGA |
| 32 | CTATACTCATGCCCCCCGGGTGCAGGTAGCAACCACAAAG |
| 33 | GTCGACTCTAGAGGATCCCCTATGTGGCGTTGGGTGCAGC |
| 35 | CATCTATACTCATGCCCCCCATGAAACCGACCACCATCTC |
| 36 | ACGGTTTCTACCAGCCACTCggcGCCTTCAATTTTGACCATGT |
| 37 | ACATGGTCAAAATTGAAGGCgccGAGTGGCTGGTAGAAACCGT |
| 38 | TGTGGTTGCTACCTGCACCCTTAATGGAAACTGTGTTCTT |
| 39 | ACTCAACTCGTCGGGATATT |
| 40 | TTCATCAGCAAAGAGTTTGG |
| 41 | TCCGCGCGGCTGTGCGGCAG |
| 42 | TGTGGTTCCAGAACCCGCTGAA |
| 43 | TTAATGGAAACTGTGTTCTT |

### (2) Production of a microorganism into which a glycine cleavage system is introduced

A vector for overexpressing the gcvPTH gene derived from *Mycobacterium smegmatis* in the ATCC13032 ΔpanB::panB(EC, G116A) strain constructed in Example 1(1) was constructed. Specifically, using genomic DNA of *Corynebacterium glutamicum* ATCC13032 as a template, a promoter fragment of the lysC gene (PlysC) was obtained, and using genomic DNA of *Mycobacterium smegmatis* ATCC 19420 (KCTC 9108) as a template, gene fragments of gcvP, gcvT, and gcvH were obtained through PCR with primer combinations of SEQ ID NOs: 9 and 10, SEQ ID NOs: 11 and 12, SEQ ID NOs: 13 and 14, and SEQ ID NOs: 15 and 16, respectively. PCR was performed by repeating 25 cycles of denaturation at 95°C for 30 seconds, annealing at 55°C for 30 seconds, and polymerization at 72°C for 3 minutes, and as a result, the PlysC (242 bp) DNA fragment and the DNA fragments of the gcvP, gcvT, and gcvH genes were obtained. The pECCG117 vector (Korean Patent Registration No. 10-0057684, No. 10-1992-0008383), which had been treated with the restriction enzyme BamHI and heat-treated at 65°C for 20 minutes, and the DNA fragments obtained above (PlysC, gcvP, gcvT, and gcvH) were cloned at a molar ratio of 2:1:1:1:1 using an Infusion Cloning Kit from TaKaRa according to the provided manual, thereby obtaining a pECCG117_PlysC-gcvPTH(M. sm) vector. The vector was transformed into the ATCC13032_ΔpanB::panB(EC, G116A) strain through electroporation to obtain a strain overexpressing GcvP, GcvT, and GcvH derived from *Mycobacterium smegmatis*, which was named ATCC13032_ΔpanB::panB(EC, G116A)_pECCG117_PlysC-gcvPTH (M.sm). The primer sequences used in the above Example are as shown in Table 2 below.

**[Table 2]**

| Primer name | Nucleic acid sequence |
|---|---|
| SEQ ID NO: 9 | ACGGTATCGATAAGCTTGATTTCAGGGTAGTTGACTAAAG |
| SEQ ID NO: 10 | GCTTTTGGCTGGTCGGACAtCTTTGTGCACCTTTCGATCT |
| SEQ ID NO: 11 | AGATCGAAAGGTGCACAAAGaTGTCCGACCAGCCAAAAGC |
| SEQ ID NO: 12 | TGCAGCAGCTCGTCACTCAtTCAGGCGAACGCCTCCACCG |
| SEQ ID NO: 13 | CGGTGGAGGCGTTCGCCTGAaTGAGTGACGAGCTGCTGCA |
| SEQ ID NO: 14 | TCGGCTGGGATCTCGCTCAtCTATCGCGTGCTCGGTGTGA |
| SEQ ID NO: 15 | TCACACCGAGCACGCGATAGaTGAGCGAGATCCCAGCCGA |
| SEQ ID NO: 16 | CCGGGCTGCAGGAATTCGATTCACTCGGTGACGACCGCGC |

### (3) Evaluation of Pantothenic Acid Production Ability

To confirm the pantothenic acid productivity of the mutant strains obtained in the above Examples, the productivity was measured by the following method. A parent strain and the mutant strain were inoculated into a 250 ml corner-baffle flask containing 25 ml of production medium, and shaking culture was performed at 32°C for 48 hours at 200 rpm. Subsequently, the culture solution was centrifuged at 20,000 rcf for 10 minutes, and the collected supernatant was diluted 10-fold with TDW (triple distilled water) and subjected to HPLC analysis to measure the pantothenic acid concentration.

### <Production Medium>

Glucose 10%, beta-alanine 0.5%, yeast extract 0.4%, ammonium sulfate 1.5%, potassium phosphate monobasic 0.1%, magnesium sulfate heptahydrate 0.05%, ferrous sulfate heptahydrate 10 mg/l, manganese sulfate monohydrate 6.7 mg/l, biotin 50 µg/l, thiamine·HCl 100 µg/l, pH 7.2

**[Table 3]**

| Strain | Pantothenic acid concentration (g/L) | L-gly concentration (g/L) | Increase in pantothenic acid concentration compared to control |
|---|---|---|---|
| Parent strain (ATCC13032_ΔpanB: :panB(EC, G116A)) | 1.6 | 0.5 | - |
| ATCC13032_ΔpanB:: panB(EC, G116A)_ pECCG117_PlysC-gcvPTH (M.sm) | 2.8 | 0.1 | 75% |

As a result, as shown in Table 3, the parent strain *Corynebacterium glutamicum* ATCC13032_ΔpanB::panB(EC, G116A) strain, produced about 1.6 g/L of pantothenic acid, but the ATCC13032_ΔpanB::panB(EC, G116A)_pECCG117_PlysC-gcvPTH(M.sm) strain with an enhanced glycine cleavage system produced pantothenic acid at a concentration of about 2.8 g/L, showing an excellent effect of improving pantothenic acid production ability.

### Example 2. Screening and Selection of Lipoate Protein Ligase Gene

In order to construct a microorganism of the genus *Corynebacterium* having enhanced lipoate protein ligase A (LplA) activity, lipoate protein ligase candidate genes derived from various microorganisms were selected, and the results are shown in Table 4 below.

**[Table 4]**

| | Strain | Primer SEQ ID NO | Vector |
|---|---|---|---|
| 1 | *Escherichia coli* (*Escherichia coli* MG1655 (ATCC 700926)) | PlysC-SEQ ID NOs: 17, 18 LpIA(EC)-SEQ ID NOs: 19, 20 | pECCG117-PlysC-gcvPTH(M.sm)_ PlysC-IplA(EC) |
| 2 | *Bacillus subtilis* (*Bacillus subtilis* ATCC 6051) | PlysC-SEQ ID NOs: 17, 21 LpIA(BS)-SEQ ID NOs: 22, 23 | pECCG117-PlysC-gcvPTH(M.sm)_ PlysC-IplA(BS) |
| 3 | *Corynebacterium glutamicum* (*Corynebacterium glutamicum* ATCC13032) | PlysC-SEQ ID NOs: 17, 24 LpIA(CG)-SEQ ID NOs: 25, 26 | pECCG117-PlysC-gcvPTH(M.sm)_ PlysC-IplA(CG) |
| 4 | *Serratia marcescens (Serratia marcescens* ATCC13880 (KCTC42171)) | PlysC-SEQ ID NOs: 17, 27 IplA(SM)-SEQ ID NOs: 28, 29 | pECCG117- PlysC-gcvPTH(M.sm)_ PlysC-IplA(SM) |

**[Table 5]**

| Primer name | Nucleic acid sequence |
|---|---|
| 17 | GGGGGATCCACTAGTTCTAGTTCAGGGTAGTTGACTAAAG |
| 18 | AGCAGGCGTAATGTGGACATCTTTGTGCACCTTTCGATCT |
| 19 | AGATCGAAAGGTGCACAAAGATGTCCACATTACGCCTGCT |
| 20 | CGCGGTGGCGGCCGCTCTAGCTACCTTACAGCCCCCGCCA |
| 21 | TGATTGTCTATAAATAACATCTTTGTGCACCTTTCGATCT |
| 22 | AGATCGAAAGGTGCACAAAGATGTTATTTATAGACAATCA |
| 23 | CGCGGTGGCGGCCGCTCTAGTTAGTAAATCAGATCAAGGA |
| 24 | AGCTCAAAATGGTTATTCATCTTTGTGCACCTTTCGATCT |
| 25 | AGATCGAAAGGTGCACAAAGATGAATAACCATTTTGAGCT |
| 26 | CGCGGTGGCGGCCGCTCTAGCTATTGAACTCGCTTAGTCC |
| 27 | AGTAAACGCAAACTGTTCATCTTTGTGCACCTTTCGATCT |
| 28 | AGATCGAAAGGTGCACAAAGATGAACAGTTTGCGTTTACT |
| 29 | CGCGGTGGCGGCCGCTCTAGCTAGCGGATGGTATCGCTCA |

### Example 3. Preparation of Microorganism of the Genus Corynebacterium with Enhanced Lipoate Protein Ligase Activity

PCR was performed using the primer sequences shown in Table 4 and Table 5 to amplify a DNA fragment encoding lipoate protein ligase, using the genome of each of the obtained microorganisms as a template. PCR was conducted using PfuUltra^{™} high-fidelity DNA polymerase (Stratagene), with PCR conditions consisting of 30 cycles of denaturation at 95°C for 30 seconds, annealing at 55°C for 30 seconds, and polymerization at 72°C for 2 minutes, thereby obtaining a polynucleotide fragment encoding a lipoate protein ligase derived from each microorganism. In addition, using *Corynebacterium glutamicum* 13032 genomic DNA as a template, PCR was performed in the same manner as described above using combinations of primers of SEQ ID NOs: 17 and 18, 17 and 21, 17 and 24, and 17 and 27 to obtain *Corynebacterium glutamicum*-derived lysC promoter DNA fragments (PlysC) for the expression of each lipoate protein ligase (from *Escherichia coli, Bacillus subtilis, Corynebacterium glutamicum,* and Serratia marcescens). After treating the pECCG117_PlysC-gcvPTH (M.sm) vector with the restriction enzyme Xbal followed by heat treatment at 65°C for 20 minutes, the vector and the DNA fragments (each IplA gene and each PlysC promoter) were cloned using a TaKaRa Infusion Cloning Kit according to the provided manual at a molar ratio (M) of 2:1:1, thereby obtaining a total of four types of vectors (Table 4).

The four types of constructed vectors were transformed into the ATCC13032 ΔpanB::panB(EC, G116A) strain constructed in Example 1(1) by electroporation to construct strains expressing each microorganism-derived lipoate protein ligase.

### Example 4. Investigation of Pantothenic Acid Production ability of Microorganism of the Genus Corynebacterium with Enhanced Lipoate Protein Ligase Activity

To confirm the pantothenic acid productivity of the strains constructed in Example 3, measurements were performed according to the following method. Specifically, a parent strain and the strains were inoculated into a 250 ml corner-baffle flask containing 25 ml of production medium, followed by shaking culture at 32°C for 48 hours at 200 rpm. After centrifuging the culture medium at 20,000 rcf for 10 minutes, the collected supernatant was diluted 10-fold with TDW (triple distilled water), and the pantothenic acid production ability was analyzed via HPLC. The results are shown in Table 6 below.

### <Production Medium>

Glucose 10%, beta-alanine 0.5%, yeast extract 0.4%, ammonium sulfate 1.5%, potassium phosphate monobasic 0.1%, magnesium sulfate heptahydrate 0.05%, ferrous sulfate heptahydrate 10 mg/l, manganese sulfate monohydrate 6.7 mg/l, biotin 50 µg/l, thiamine·HCl 100 µg/l, pH 7.2

**[Table 6]**

| Strain | Pantothenic acid concentration (g/L) | L-Gly concentration (g/L) | Increase in pantothenic acid concentration compared to control |
|---|---|---|---|
| Parent strain (ATCC13032_ΔpanB: :panB(EC, G116A)_pECCG117_ PlysC-gcvPTH(M. sm)) | 2.5 | 0.2 | |
| ATCC13032_ΔpanB:: panB(EC, G116A)_pECCG117_ PlysC-gcvPTH(M. sm)-PlysC-lplA(EC) | 2.2 | 0.2 | -12.00% |
| ATCC13032_ΔpanB:: panB(EC, G116A)_pECCG117_ PlysC-gcvPTH(M. sm)-PlysC-lplA(BS) | 3.1 | 0.0 | 24.00% |
| ATCC13032_ΔpanB:: panB(EC, G116A)_pECCG117_ PlysC-gcvPTH(M. sm)-PlysC-lplA(CG) | 2.5 | 0.2 | 0.00% |
| ATCC13032_ΔpanB:: panB(EC, G116A)_pECCG117_ PlysC-gcvPTH(M. sm)-PlysC-IplA(SM) | 1.8 | 0.3 | -28.00% |

As a result, as shown in Table 6, the parent strain *Corynebacterium glutamicum* ATCC13032_ΔpanB::panB(EC, G116A)_pECCG117_PlysC-gcvPTH (M.sm), produced about 2.5 g/L of pantothenic acid, but ATCC13032_ΔpanB::panB(EC, G116A)_pECCG117_PlysC-gcvPTH(M. sm)-PlysC-lplA(BS), a strain expressing LlplA derived from *Bacillus subtilis*, showed the highest pantothenic acid productivity at 3.1 g/L.

From the above description, a person skilled in the art to which the present invention belongs may understand that the present invention may be implemented in other specific forms without changing its technical idea or essential characteristics. In this regard, the embodiments described above should be understood as illustrative in all respects and not restrictive. The scope of the present invention should be interpreted such that all modifications or variations derived from the meaning and scope of the following claims and equivalent concepts thereof, rather than the detailed description above, are included in the scope of the present invention.

## Claims

1. A microorganism of the genus *Corynebacterium* producing pantothenic acid or pantoic acid, in which lipoate protein ligase activity is enhanced.

2. The microorganism of the genus *Corynebacterium* according to claim 1, wherein the lipoate protein ligase comprises an amino acid sequence of SEQ ID NO: 1 or an amino acid sequence having 90% or more sequence identity therewith.

3. The microorganism of the genus *Corynebacterium* according to claim 1, wherein the lipoate protein ligase is an LplA protein.

4. The microorganism of the genus *Corynebacterium* according to claim 1, wherein the microorganism is one into which lipoate protein ligase derived from *Bacillus subtilis* or a polynucleotide encoding the same is introduced.

5. The microorganism of the genus *Corynebacterium* according to claim 1, wherein the microorganism of the genus *Corynebacterium* has enhanced glycine cleavage system activity.

6. The microorganism of the genus *Corynebacterium* according to claim 5, wherein the glycine cleavage system comprises one or more subunits selected from the group consisting of GcvP, GcvT, and GcvH polypeptides.

7. The microorganism of the genus *Corynebacterium* according to claim 5, wherein the glycine cleavage system is derived from *Mycobacterium smegmatis*.

8. The microorganism of the genus *Corynebacterium* according to claim 1, wherein the microorganism is *Corynebacterium glutamicum*.

9. A method for producing pantothenic acid or pantoic acid, comprising culturing the microorganism of the genus *Corynebacterium* according to any one of claims 1 to 8 in a medium.

10. The method for producing pantothenic acid or pantoic acid according to claim 9, further comprising recovering pantothenic acid or pantoic acid from the medium or the microorganism resulting from the culture.

11. A composition for producing pantothenic acid or pantoic acid, comprising the microorganism of the genus *Corynebacterium* according to any one of claims 1 to 8.

12. Use of the microorganism of the genus *Corynebacterium* according to any one of claims 1 to 8 for producing pantothenic acid or pantoic acid.
